## Europäisches Patentamt

(19) ))) **European Patent Office**

**Office européen des brevets**

(11) Publication number: **0 134 671**
**B1**

(12) # EUROPEAN PATENT SPECIFICATION

(45) Date of publication of patent specification: **15.04.87**

(51) Int. Cl.⁴: **A 61 F 5/46**

(21) Application number: **84304889.3**

(22) Date of filing: **18.07.84**

(54) Contraceptive membrane ring.

(30) Priority: **19.07.83 US 515343**

(43) Date of publication of application:
**20.03.85 Bulletin 85/12**

(45) Publication of the grant of the patent:
**15.04.87 Bulletin 87/16**

(84) Designated Contracting States:
**AT BE CH DE FR GB IT LI LU NL SE**

(56) References cited:
**DE-C- 355 944**
**DE-C- 463 331**
**DE-C- 574 471**
**US-A-2 145 057**
**US-A-3 015 598**
**US-A-3 128 767**
**US-A-3 216 422**
**US-A-3 786 807**
**US-A-3 983 874**
**US-A-4 198 976**
**US-A-4 200 090**
**US-A-4 300 544**
**US-A-4 369 773**

(73) Proprietor: **THE POPULATION COUNCIL, INC.**
**1230 York Avenue**
**New York New York 10021 (US)**

(72) Inventor: **Luukkainen, Tapani**
**Alkutie 79**
**SF-Helsinki 66 (FI)**

(74) Representative: **Ford, Michael Frederick et al**
**MEWBURN ELLIS & CO. 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Although contraceptives which depend on metabolic and hormone mechanisms have become increasingly popular in recent years, there is a current interest in and return to the use of barrier contraceptives by women. For centuries, women have been using barrier contraceptives, such as diaphragms and sponges, and in recent years, numerous changes and improvements have been made to make them safer and more convenient to use. For example, see U.S. Patent Nos. 4,300,544; 3,983,874; 4,200,090; 4,198,976; 3,786,807 and 3,015,598; DE—C—463331 discloses a pessary comprising a pessary cap in the bottom of which a disposable, rigid, porous insert is provided which can be impregnated with disinfectant or the like.

The major drawbacks with even the improved diaphragms described in the recent patent literature are that a diaphragm is often difficult to use neatly and must be individually fitted by a doctor. The diaphragms are often poorly retained in the vagina which decreases the time period during which they can be effectively worn and conventional diaphragms allow direct contact between the spermicide and the vaginal mucosa.

The present membrane ring overcomes the problems often encountered with conventional diaphragms and provides women with a novel barrier contraceptive which is neat and easy to use for longer periods of time, limits absorption of spermicide by the vaginal mucosa and does not require individual fitting.

The membrane ring is a barrier contraceptive device designed to cover the cervical os and thereby prevent the entry of sperm into the uterus. It comprises a flexible ring having a top and buttom surface;

a sperm impermeable flexible membrane stretched across and attached to the bottom surface of the ring so as to prevent the entry of sperm into the uterus;

a disposable flexible insert impregnated with a spermicide and made of an absorbent material, the insert having a diameter slightly larger than the inner diameter of the ring which allows the insert to fit snugly within the inner circumference of the ring, the insert being positioned above but resting on the sperm impermeable flexible membrane; and

a flexible permeable cover positioned directly on top of the insert and pressing against the inner circumference of the ring and having a diameter at least equal to the inner diameter of the ring which holds the insert in place on the flexible membrane. The insert and cover may be combined.

Embodiments of this invention will now be described with reference to the drawings, in which:

Figure 1 is a top plan view of contraceptive membrane ring constructed in accordance with the teaching of the invention.

Figure 2 is a cross sectional view on the line 2, 2 through the membrane ring of Figure 1, looking in the direction of the arrows.

Figure 3 is a bottom plan view of the contraceptive membrane ring.

Figure 4 is a top plan view of the insert.

Figure 5 is a top plan view of the flexible cover.

Figure 6 is a top plan view of a combined cover/insert.

Figure 7 is an exploded view of the contraceptive membrane ring of Figure 1.

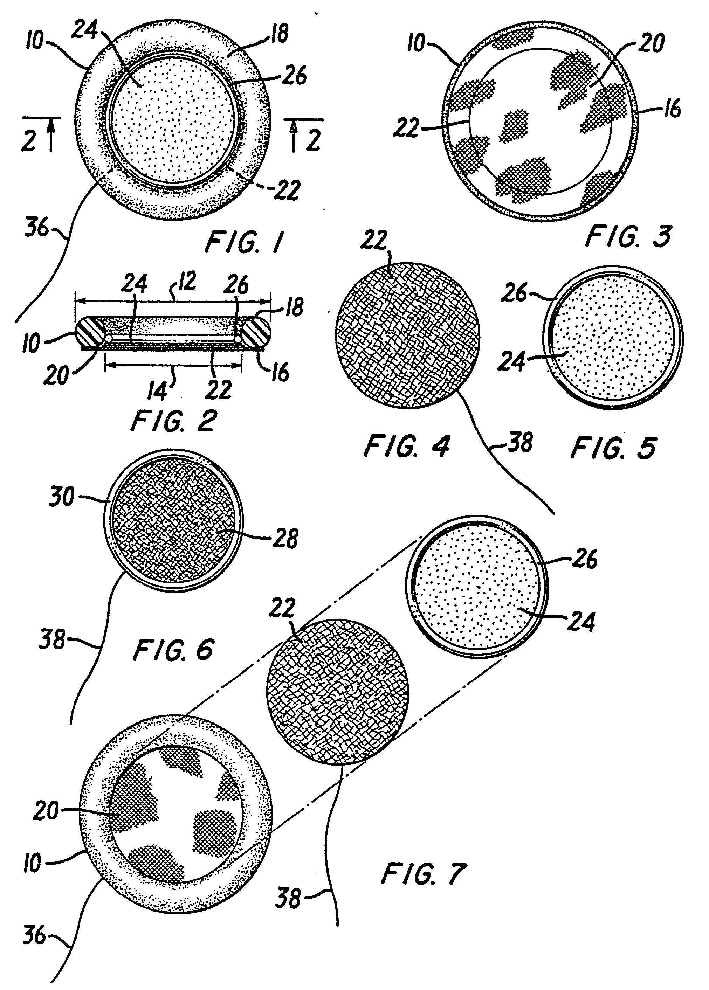

The membrane ring as illustrated in Figure 1 generally comprises the flexible ring 10, which has a generally circular shape. The ring can be constructed from any suitable elastomer which is stable, flexible and non-irritating, for example:

Thermosetting organopolysiloxanes to be vulcanized with peroxide curing catalysts, e.g. benzoyl peroxide or di-p-chlorobenzoyl peroxide at temperatures of about 150—200°C and requiring a heat aftertreatment, e.g. those described in U.S. Patent Nos. 2,541,137; 2,723,966; 2,863,846; 2,891,188 and 3,022,951.

Hydroxyl-terminated organopolysiloxanes of the RTV (room temperature vulcanizing) type which harden to elastomers at room temperature after the addition of cross-linking agents in the presence of curing catalysts and under atmospheric humidity. Typical curing catalysts are metallic salts of carboxylic acids, preferably tin salts, e.g. tin (II) octoate and tin (II)-2-ethylhexanoate.

Single-component silicone rubber compositions which are cured at room temperature under atmospheric humidity without any further additives. These single component compositions contain primarily organopolysiloxanes with two terminal-positioned hydrolyzable acyloxy groups, e.g. acetoxy; the acyloxy groups are hydrolyzed under atmospheric humidity to form trifunctional siloxane units which cross-link the polymer into a cured elastomer. Such organopolysiloxanes are described, e.g. in U.S. Patent Nos. 2,927,907 and 3,035,016 and in British Patent Nos. 798,669 and 804,199.

Two-component dimethylpolysiloxane compositions, platinum-catalyzed at room temperature or under slightly elevated temperature and capable of addition cross-linking. The preferred elastomers for use in the membrane ring are polydimethylsiloxanes such as medical grade Silastic rubber. Polyurethanes, natural and synthetic rubbers, copolymers of ethylene and vinyl acetate can also be used.

The flexible ring 10 should be of suitable rigidity such that the force needed to cause the inside faces of the ring to touch in response to pressure across the diameter is in the range of 8.9—22.2 N (2—5 lbs.), 13.3—17.8 N (3—4 lbs.) of force being preferred. In order that the membrane ring will be better tolerated and retained longer in place over the cervical os, the ring 10 has a cross-sectional diameter as shown in Figure 2 of about 5—10 mm but preferably 6—8 mm. The ring has an outer diameter 12 of about 50—65 mm, preferably 54—58 mm and an inner diameter 14 of about 30—55 mm, preferably 38—46 mm. The ring has a bottom surface 16 and a top surface 18. The ring can be made by any known means, for example the method of manufacture described in U.S. Patent No. 4,292,965 can be used.

A flexible membrane 20 made of sperm impermeable material is stretched across and attached to the bottom surface 16 of the ring to form an essentially flat, sperm impermeable barrier having a top and bottom surface. The flexible membrane 20 can be of any stable, non-irritating, flexible polymer, for example those described above for use in construction of the ring. The membrane can be attached to the ring by any conventional sealing means such as heat sealing or adhesives.

An absorbent, flexible, disposable circular insert 22 as illustrated in Figure 4 impregnated with an effective spermicide is placed on top of the flat sperm impermeable flexible membrane 20. The insert 22 has a diameter slightly larger than the inner diameter of the ring 10 and is made of a flexible material that will fold when the ring is compressed for insertion into the vaginal vault but will spring back into place when the pressure on the ring is released. Suitable materials for construction of the insert 22 would include for example cloth, an elastomer, synthetic foams such as polyurethane or collagen and absorbent paper of suitable porosity. The insert 22 is about 30—55 mm in diameter but is about 38—46 mm in diameter in the preferred embodiment.

Suitable spermicides for impregnating the insert 22 include, for example, but are not limited to dodecaethylene glycol monolaurate p-methanyl-phenyl polyoxyethylene (8.8) ether (TS-88), methoxypolyoxyethylene glycol 550 laurate, Di-isobutylphenoxy polyethoxy ethanol, polyoxy-ethylenenonyl phenol, phenyl mercuric acetate (PMA), nonoxynol-9, polyethyleneglycol of mono-isoctyl phenol ether, polysaccharidepolysulfuric acid ester, chloramine, sodium dichlorosul-phamidobenzoate, gossypol and its derivatives for example acetates and methyl ethers and benzethonium chloride.

In one embodiment of the invention, the circular insert 22 is held in place on the top surface of the membrane by a flexible permeable cover 24 having a diameter at least equal to the diameter of the insert 22, preferably 38—46 mm. Like the insert 22, the cover 24 must be flexible enough to return to its original shape after being folded and compressed during insertion of the membrane ring and must be held firmly in place on top of the insert and pressing against the inner circumferences of the ring. This can be accomplished, for example, by fitting the cover 24 with a firm but flexible rim 26 about 2—3 mm in diameter. The rim 26 is separately fastened to the circumferences of the cover by heat sealing, adhesive or any other conventional methods. In the alternative, the rim 26 is formed or molded by conventional means as an integral part of the cover. The cover 24 and rim 26 may be made from any medically suitable flexible material. The rim can also be made from a stable, flexible, non-irritating metal or plastic spring.

In an alternate preferred embodiment, the insert 22 and the cover 24 are combined into one disposable, flexible, circular cover/insert 28 which is made of the same absorbent material as insert 22. The cover/insert 28 has a diameter at least equal to the diameter of the ring and must be held firmly in place on top of the flexible membrane 20 and pressing against the inner circumference of the ring. This can be accomplished, for example, by fitting the cover/insert with a flexible rim 30 to hold it firmly within the inner circumference of the ring 10. The rim 30 can be formed in the same manner and of the same material as rim 26. The combined cover/insert 28 is impregnated with a suitable spermicide.

To position the membrane ring for use, it is folded by applying pressure to both sides of the ring 10 and is placed in the vagina in an open position posterior to the introitus in a manner such that the cover 24 and/or cover/insert 28 covers the cervical os. After use, the ring membrane may be removed in the same manner as a diaphragm.

The membrane ring may be fitted with a string or loop 36 to facilitate removal from the vaginal vault after use. The disposable inserts 22 or cover/insert 28 may also be fitted with a string or loop 38 to facilitate removal and disposal of the inserts after use. After removing the disposable inserts 22 or cover/insert 28 from the ring membrane they are discarded and, the ring membrane is cleaned and stored or fitted with a new insert and repositioned in the vaginal vault.

The disposable insert 22 or cover/insert 28 are impregnated with a spermicide and can be supplied already impregnated, for example, individually wrapped in foil or plastic or a larger supply may be stacked and packaged in a sealed wide mouth jar or bottle for easy storage and dispensing.

The size of the membrane ring allows it to fit most women and it need not be individually fitted as with a conventional diaphragm. Because child birth usually affects the size of the vaginal vault, the ring is preferably made in two overall diameters; that is, about 50 mm for nulliparous women and about 60 mm for parous women. Because of the design of the ring, the membrane ring is easily retained in the vagina positioned over the cervical os without being readily displaced. It can be left in place for at least twelve hours and it is anticipated that it will remain effective as a contraceptive even after longer periods of use. Unlike a diaphragm, the spermicide in the membrane ring is actually impregnated in the insert 22 or cover/insert 28 and therefore does not quickly dissipate through leakage as in a conventional diaphragm-spermicide combination.

The design of the membrane ring also serves to remove the spermicide from direct contact with the vaginal mucosa which limits absorption of the spermicide through the mucosa.

## Claims

1. A contraceptive membrane ring designed to cover the cervical os which comprises a flexible ring (10) having a top (18) and bottom (16) surface, a sperm impermeable flexible membrane (20) stretched across and attached to the bottom surface (16) of the ring, a disposable flexible insert (22) resting on the sperm impermeable membrane (20), the insert being made of an absorbent material and having a diameter slightly larger than the inner diameter (14) of the ring (10), the insert further being impregnated with a spermicide, and a flexible permeable cover (24) positioned directly on top of the insert (22) and pressing against the inner circumference of the ring, the cover having a diameter at least equal to the inner diameter (14) of the ring.

2. A membrane ring as described in claim 1 wherein the cover (24) and insert (22) are combined in one disposable flexible cover/insert (28) which comprises a flexible insert preimpregnated with a spermicide.

3. A membrane ring as described in claim 2 wherein the cover/insert (28) is fitted with a flexible rim (30) to hold it firmly within the inner circumference of the ring.

4. A membrane ring as described in claim 1 wherein the cover (24) is fitted with a flexible rim (26) to hold it firmly within the inner circumference of the ring.

5. A membrane ring as described in claim 1 wherein the ring is fitted with a means (36) to facilitate removal.

6. A membrane ring as described in claim 2 wherein the ring is fitted with a means to facilitate removal.

7. A membrane ring as described in claim 1 wherein the ring (10) has a cross sectional diameter of about 5—10 mm, an outer diameter (12) of about 50—65 mm and an inner diameter (14) of about 30—55 mm; the insert (22) is about 30—55 mm in diameter; and the cover (24) has a flexible rim (26) about 2—3 mm in diameter.

8. A membrane ring as described in claim 2 wherein the ring has a cross sectional diameter of about 5—10 mm, an outer diameter of about 50—65 mm and an inner diameter of about 30—55 mm; the cover/insert (28) is about 30—55 mm in diameter and has a flexible rim (30) about 2—3 mm in diameter.

## Patentansprüche

1. Ein empfängnisverhütender Membranring zur Abdeckung des Gebärmuttermundes, umfassend einen flexiblen Ring (10) mit einer oberen (18) und einer unteren (16) Fläche, eine für Sperma undurchlässige, flexible Membran (20), die über die untere Fläche (16) des Ringes gespannt und an dieser befestigt ist, einen wegwerfbaren, flexiblen Einsatz (22), der auf der für Sperma undurchlässigen Membran (20) aufliegt, aus einem absorbierenden Material besteht und einen Durchmesser aufweist, der etwas größer ist als der innere Durchmesser (14) des Ringes (10), welcher Einsatz (22) weiters mit einem Spermizid imprägniert ist, und eine flexible, durchlässige Abdeckung (24), die direkt oben auf dem Einsatz (22) in Stellung gebracht ist und gegen den Innenumfang des Ringes drückt, wobei der Durchmesser der Abdeckung wenigstens gleich dem Innendurchmesser (14) des Ringes ist.

2. Ein Membranring nach Anspruch 1, worin die Abdeckung (24) und der Einsatz (22) zu einer wegwerfbaren, flexiblen Einheit aus Abdeckung/Einsatz 28 kombiniert sind, die einen flexiblen, mit Spermizid vorimprägnierten Einsatz umfaßt.

3. Ein Membranring nach Anspruch 2, worin die Einheit aus Abdeckung/Einsatz 28 mit einem flexiblen Rand (30) versehen ist, um sie fest innerhalb des Innenumfangs des Ringes in Stellung zu halten.

4. Ein Membranring nach Anspruch 1, worin die Abdeckung (24) mit einem flexiblen Rand (26) versehen ist, um sie fest innerhalb des Innenumfangs des Ringes in Stellung zu halten.

5. Ein Membranring nach Anspruch 1, worin der Ring mit einer Einrichtung (36) zur Erleichterung seiner Entfernung versehen ist.

6. Ein Membranring nach Anspruch 2, worin der Ring mit einer Einrichtung zur Erleichterung seiner Entfernung versehen ist.

7. Ein Membranring nach Anspruch 1, worin der Ring (10) einen Querschnittdurchmesser von etwa 5—10 mm, einen Außendurchmesser (12) von etwa 50—65 mm und einen Innendurchmesser (14) von etwa 30—35 mm aufweist, der Einsatz (22) einen Durchmesser von etwa 30—35 mm besitzt und die Abdeckung (24) mit einem flexiblen Rand (26) versehen ist, dessen Durchmesser etwa 2—3 mm beträgt.

8. Ein Membranring nach Anspruch 2, worin der Ring einen Querschnittdurchmesser von etwa 5—10 mm, einen Außendurchmesser von etwa 50—65 mm und einen Innendurchmesser von etwa 30—35 mm aufweist, die Einheit aus Abdeckung/Einsatz (28) einen Durchmesser von etwa 30—35 mm besitzt und mit einem flexiblen Rand (30) versehen ist, der einen Durchmesser von etwa 2—3 mm aufweist.

## Revendications

1. Anneau contraceptif à membrane destiné à recouvrir l'os cervical, qui comprend un anneau flexible (10) ayant une surface de dessus (18) et une surface de dessous (16), une membrane (20) flexible imperméable au sperme étirée au travers de la surface de dessous (16) de l'anneau et qui y est attachée, un organe d'insertion (22) flexible, jetable reposant sur la membrane (20) imperméable au sperme, l'organe d'insertion étant réalisé en un matériau absorbant et ayant un diamètre légèrement plus grand que le diamètre interne (14) de l'anneau (10), l'organe d'insertion étant de plus imprégné d'un spermicide, et une coiffe (24) perméable flexible placée directement sur le dessus de l'organe d'insertion (22) et pressant contre la circonférence interne de l'anneau, la coiffe ayant un diamètre au moins égal au diamètre interne (14) de l'anneau.

2. Anneau à membrane selon la revendication 1, dans lequel la coiffe (24) et l'organe d'insertion (22) sont combinés pour former un ensemble (28) organe d'insertion/coiffe flexible jetable qui comprend un organe d'insertion flexible préimprégné d'un spermicide.

3. Anneau à membrane selon la revendication 2, dans lequel l'ensemble organe d'insertion/ coiffe (28) est muni d'un rebord flexible (30) pour le maintenir solidement à l'intérieur de la circonférence interne de l'anneau.

4. Anneau à membrane selon la revendication 1, dans lequel la coiffe (24) est munie d'un rebord flexible (26) pour la maintenir solidement à l'intérieur de la circonférence interne de l'anneau.

5. Anneau à membrane selon la revendication 1, dans lequel l'anneau est muni de moyens (36) pour faciliter son enlèvement.

6. Anneau à membrane selon la revendication 2, dans lequel l'anneau est muni de moyens pour faciliter son enlèvement.

7. Anneau à membrane selon la revendication 1, dans lequel l'anneau (10) présente un diamètre en coupe transversale d'environ 5—10 mm, un diamètre externe (12) d'environ 50—65 mm et un diamètre interne (14) d'environ 30—55 mm; l'organe d'insertion (22) a un diamètre d'environ 30—55 mm, et la coiffe (24) présente un rebord flexible (26) d'environ 2—3 mm de diamètre.

8. Anneau à membrane selon la revendication 2, dans lequel l'anneau présente un diamètre en coupe transversale d'environ 5—10 mm, un diamètre externe d'environ 50—65 mm et un diamètre interne d'environ 30—55 mm; l'ensemble organe d'insertion/coiffe (28) a un diamètre d'environ 30—55 mm et présente un rebord flexible (30) d'environ 2—3 mm de diamètre.

FIG. 1

FIG. 2

FIG. 3

FIG. 4

FIG. 5

FIG. 6

FIG. 7